# EUROPEAN PATENT APPLICATION

(11) **EP 2 679 268 A1**
(43) Date of publication of application: **01.01.2014**
(21) Application number: 12250125.7
(22) Date of filing: 27.06.2012
(51) Int. Cl.: A61M 25/01, A61B 17/34, A61M 5/158, A61M 25/00, A61M 39/02

(54) **Infusion catheter system**

(71) Applicant: Peak Medical Ltd., Charlbury Oxfordshire OX7 3EW (GB)
(72) Inventor: Swaddle, Gary, Castleside, Durham DH8 9QS (GB)
(74) Representative: Scullion, Juliet Patricia

(57) **Abstract**

An infusion catheter system comprises a surgical needle (30) and an infusion catheter (40). The infusion catheter has an internal bore; and the needle has a protrusion (36) extending from its proximal end (34). The protrusion is adapted to mate with the internal bore of the infusion catheter. Through this innovative connection method, the needle can take the same diameter as the catheter. Therefore the puncture marks caused in the patient will have a smaller diameter than with conventional "introducer needles", reducing pain, leakage and the chances of infection. Furthermore, the catheter may be introduced subcutaneously, further reducing the risk of infection by eliminating the risk of skin bacteria being drawn into the wound cavity.

## Description

### FIELD OF THE INVENTION

The present invention relates to a catheter system and more particularly to an infusion catheter system, a surgical needle for use within an infusion catheter system, and a method of using an infusion catheter system, in which anaesthetic (or any other suitable medicinal substance) is infused into a wound cavity.

### BACKGROUND ART

Postoperative pain management is an integral component of a patient's overall recovery from surgery. The under-treatment of postoperative pain can give rise to adverse outcomes that include thromboembolic and pulmonary complications, prolonged stay in an intensive care unit and/or in a hospital, and the onset of chronic pain.

Satisfactory acute pain management following surgery is generally achieved through the use of systemic narcotic analgesics delivered via intravenous, intramuscular or oral administration. In addition, many patients benefit from intravenous, patient-controlled anaesthesia (PCA) with narcotic analgesics, affording patients a certain amount of control over their pain.

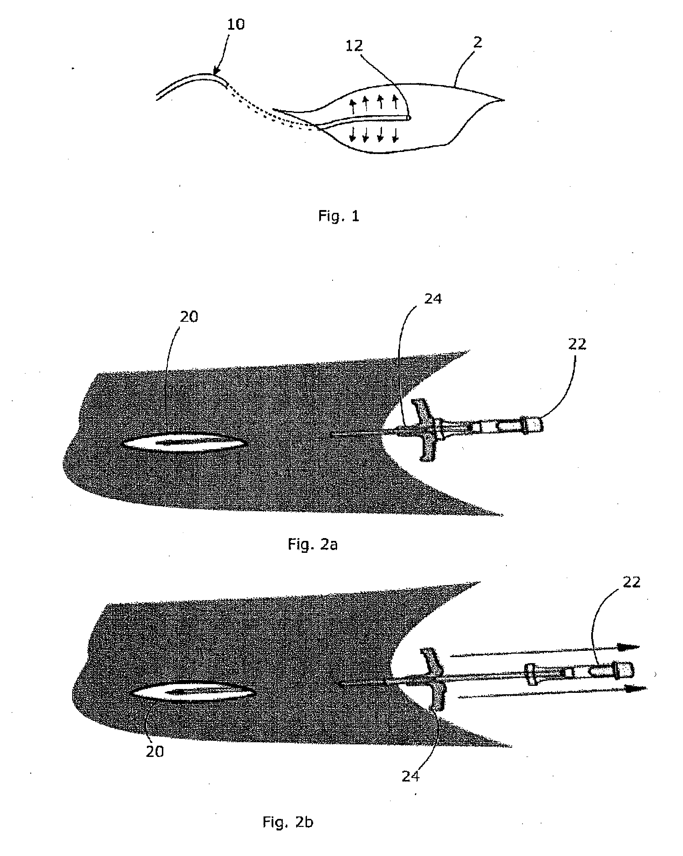

The use of systemic narcotic analgesics carries with it several unfavourable side effects. For that reason, direct instillation of local anaesthetics and/or opioids into operative sites has been proposed as a pain management technique to reduce the need for oral or parenteral postoperative narcotics. Local anaesthetics, opioids or both can be administered by an infusion pump directly into the surgical site, including intra-articular, soft tissue, and perineural locations. The perineural technique involves the percutaneous insertion of a catheter directly adjacent to the peripheral nerves supplying an affected surgical site. Local anaesthetic is then infused via the catheter providing site-specific analgesia. Figure 1 shows an example of this, where an incision 2 has been made during a surgical procedure. A catheter 10 is introduced from a site adjacent the incision, and fed through to the wound cavity. Analgesics are fed through the catheter by a pump (not illustrated) and suffuse outwards via perforations in the distal end 12.

In general, ambulatory pumps consist of an elastomeric membrane, which provides positive pressure for medicine delivery. These elastomeric pumps are either available in various unalterable combinations of pre-programmed reservoir volumes and infusion rates or incorporate a regulator within the administration line by which the clinician can increase flow rates to suit the patient's needs.

Anaesthetic agents used for local analgesia include bupivacaine, ropivacaine and levobupivacaine.

The advantages of this method of pain management are direct pain relief without the side effects of narcotics, reduction in breakthrough pain, and shorter recovery times.

Since these devices tend to be ambulatory, patients can be discharged back into the community with their catheters in place and receiving a continuous infusion of anaesthetic agent from the portable pump. However, potential complications associated with the use of pain pump systems include surgical wound infection, and leakage of medication back through the point at which the catheter enters the skin. Other reported complications involve the catheter itself, which include but are not limited to, kinking, breakage, occlusion, disconnect, poor position, migration and reduced flow. Thus, the success of an ambulatory pump system is dependent in large part on a successful and dependable connection between the pump and the catheter as well as the design of the catheter and the introduction techniques employed.

The usual method to introduce the catheter is to use an introducer needle 20 (see Figures 2a to 2d). Such needles generally consist of a sharp needle 22 and a surrounding sleeve 24, or sheath. The procedure is as follows.

The introducer needle punctures the skin all the way through to the wound cavity 20 (see Figure 2a). Once there, the central needle part 22 is removed and discarded, leaving the surrounding plastic sleeve 24 in place (see Figure 2b).

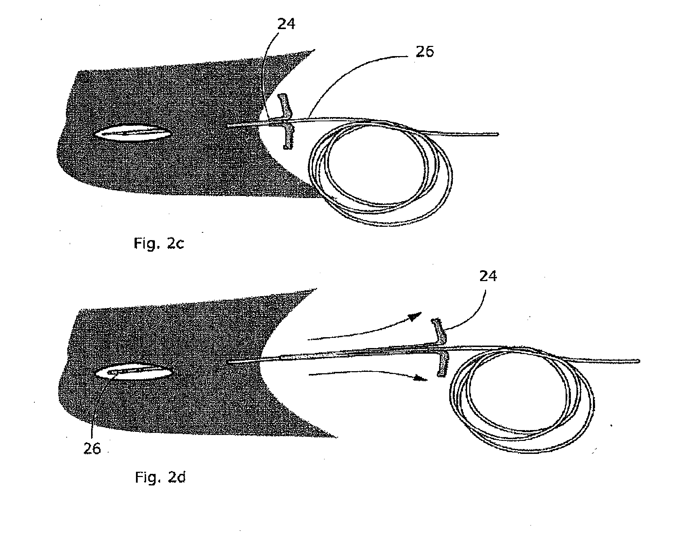

The distal end of an infusion catheter 26 is then pushed through the centre of the sleeve 24 and into the wound cavity 20 (Figure 2c). The sleeve 24 is then withdrawn away from the patient, peeled in half by the surgeon (it is designed to facilitate peeling) and discarded, leaving the catheter in-situ (Figure 2d). The catheter can then be connected to an ambulatory pump to complete the procedure.

The use of an introducer needle causes excessive trauma to surrounding tissue; because the complete device (i.e. including both the needle 22 and its surrounding sleeve 24) is greater in diameter than the catheter 26 itself, the resulting point of entry is larger than necessary. This causes problems after wound closure, causing the leakage of fluids and medicine from within the wound and increasing the likelihood of infection and movement of the catheter. In addition, the size and methodology of the introducer needle mean that it must be introduced to the patient from the outside. In the act of puncturing the skin, bacteria present on the outside of the skin are drawn inside the patient, potentially as far as the wound cavity. Infection is an uncommon, but occasionally devastating, complication of orthopaedic surgery in particular.

### SUMMARY OF THE INVENTION

Embodiments of the present invention avoid the creation of a larger entry point than necessary, and may be used in such a way which minimizes the risk of post-operative infection.

In one aspect, the present invention provides an infusion catheter system, comprising: an infusion catheter having an internal bore; and a needle having a tip at one end, and a protrusion extending from the other end. The protrusion is adapted to mate with the internal bore of the infusion catheter.

Through this innovative connection method, the needle can take the same external diameter as the infusion catheter, reducing the size of puncture marks when compared to the conventional "introducer needle" concept. This has the effect of reducing pain, leakage and the chances of infection. Accordingly, in one embodiment, the needle has an external diameter which is the same as that of the infusion catheter.

The protrusion may be barbed, to strengthen the connection between the needle and the catheter, or merely shaped and sized relative to the internal bore so as to achieve an interference fit between the two components, i.e. so that their diameters are substantially the same.

It will be apparent from the discussion above that the surgical needle has a substantially reduced diameter compared to the introducer needle. Accordingly, the protrusion from the needle has a smaller diameter still. In one embodiment, the external diameter of the protrusion is in the range from 0.25 mm to 0.75 mm. Within that range, 0.4 mm to 0.6 mm is preferred.

The infusion catheter will in general have some apertures through which medicinal liquid can escape to the patient. For example, in one embodiment the catheter has a number of perforations (or fenestrations), positioned towards its distal end. The perforations may be arranged in a helical pattern to aid even distribution of the medicine.

In further embodiments, the infusion catheter comprises a metalically reinforced section, extending perhaps along the majority of its length. The reinforcement may comprise a helically wound metal wire, or any alternative within the general knowledge of the skilled person. The infusion catheter may comprise a portion along its length which is free from said reinforcement, positioned, for example, at the catheter's proximal end. This non-reinforced section will aid the connection between the needle and the catheter, and will also provide a useful point at which the surgeon can cut to remove the needle after use.

According to a further aspect of the present invention, a surgical needle is provided for connection to an infusion catheter. The needle comprises: a tip at a first end; and a protrusion extending from a second end. The protrusion has an external diameter smaller than the diameter of the needle at said second end.

The present invention also contemplates a method of deploying an infusion catheter in a patient (human or animal) having a wound cavity. The method employs a system as set out above, wherein the protrusion of the needle has been inserted into the internal bore of the infusion catheter (for example as part of the surgical procedure or earlier during the manufacturing process). The method comprises: pulling the needle from a subcutaneous position in the wound cavity out through the skin; detaching the needle from the infusion catheter; and connecting the infusion catheter to a pump.

The needle can be detached simply by cutting the infusion catheter. In embodiments where the infusion catheter comprises at least one metallically reinforced portion and at least one non-reinforced portion, and the infusion catheter can be cut at one of the one non-reinforced portions.

### BRIEF DESCRIPTION OF THE DRAWINGS

Preferred embodiments of the present invention will now be described hereinbelow by way of example only with reference to the accompanying drawings, in which;
Figure 1 shows an infusion catheter in situ within a wound cavity;
Figures 2a to 2d represent a conventional method of deploying an infusion , catheter;
Figure 3 shows an infusion catheter system in accordance with a preferred embodiment of the present invention;
Figure 4 shows in close up of detail A of Figure 3;
Figure 5 shows a lateral sectional view (section I-I in Figure 3) through the infusion catheter of the infusion catheter system of Figure 3; and
Figures 6a and 6b represent a method of deploying an infusion catheter according to a preferred embodiment of the present invention.

### DETAILED DESCRIPTION OF THE EMBODIMENT

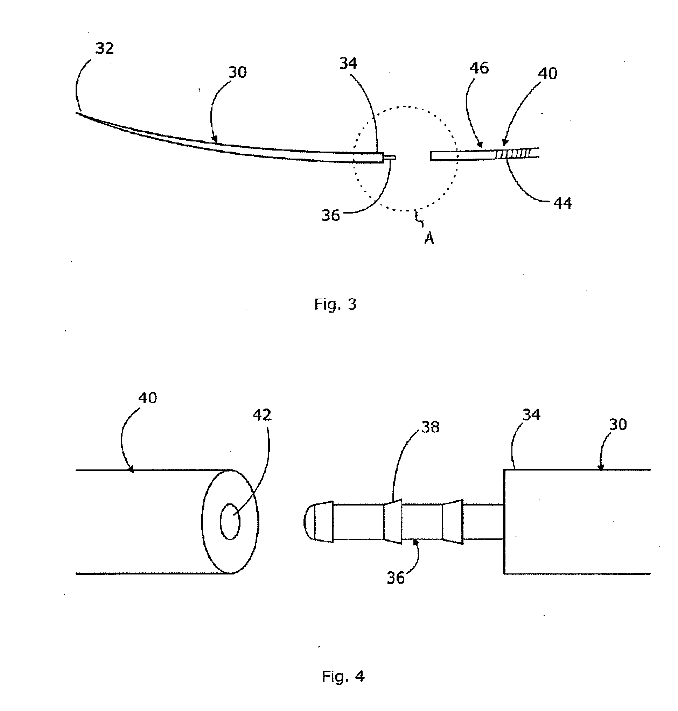

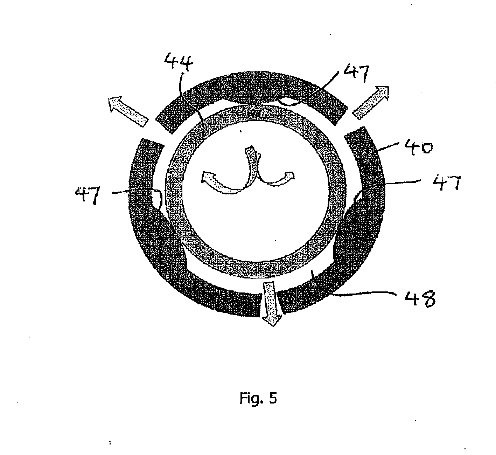

Figures 3 to 5 show an infusion catheter system according to a preferred embodiment of the present invention.

A surgical needle 30 has an elongate body, with a sharp tip 32 at a first end and a spike, or protrusion 36 at a second end 34. In the illustrated embodiment, the diameter of the needle increases smoothly from the tip 32 towards the second end 34, and the needle itself is curved (so as to be suitable for orthopaedic or laparoscopic work). However, in other embodiments the needle may be straight (as would be suitable for soft-tissue work, for example). The needle 30 may taper to the tip 32 along its entire length or along only a portion of that length.

The protrusion 36 has a narrower diameter than the needle, and in the illustrated embodiment has a number of barbs 38. In one embodiment, the protrusion 36 has a diameter *d* in a range from 0.25 mm to 0.75 mm; a diameter *d* in the range of 0.4 mm to 0.6 mm is further preferred. In another embodiment, the second end 34 of the needle has an external diameter in the range of 0.75 mm to 1.25 mm.

The needle is made of solid, surgical steel. In order to manufacture an apparatus with such high tolerances, laser milling may be used.

An infusion catheter 40 is also provided. This is a thin tube with a number of perforations at the distal end (i.e. that furthest removed from the needle 30) to allow egress of analgesic once placed inside a wound cavity. In one embodiment, the perforations are arranged in a helical pattern with regular spacing. The catheter has an internal bore 42 (or single lumen), into which the protrusion 36 is inserted prior to use, or as part of the manufacturing process. The relative diameters of the bore 42 and the protrusion 36 are carefully chosen to ensure a tight fit. In some embodiments, an interference fit may be sufficient to hold the two components together securely. In other embodiments, barbs 38 strengthen the connection.

Once placed inside the patient, kinking or collapse of the catheter can prevent analgesic from reaching the wound cavity. To mitigate against this problem, a helically wound metal wire 44, as a reinforcing element, may be provided to reinforce the wall of the catheter 40 and minimize the risk of blockage. However, in addition, at least one region 46 may be provided, at the proximal end of the catheter, in which no reinforcing wire is present. The use of this region will be explained in greater detail below.

In this embodiment the catheter 40 includes a plurality of supports 47, here ribs, which extend along a length of an inner surface of the catheter 40 and support the metal wire 44 in order to maintain a lumen 48 between an outer surface of the metal wire 44 and an inner surface of the catheter 40.

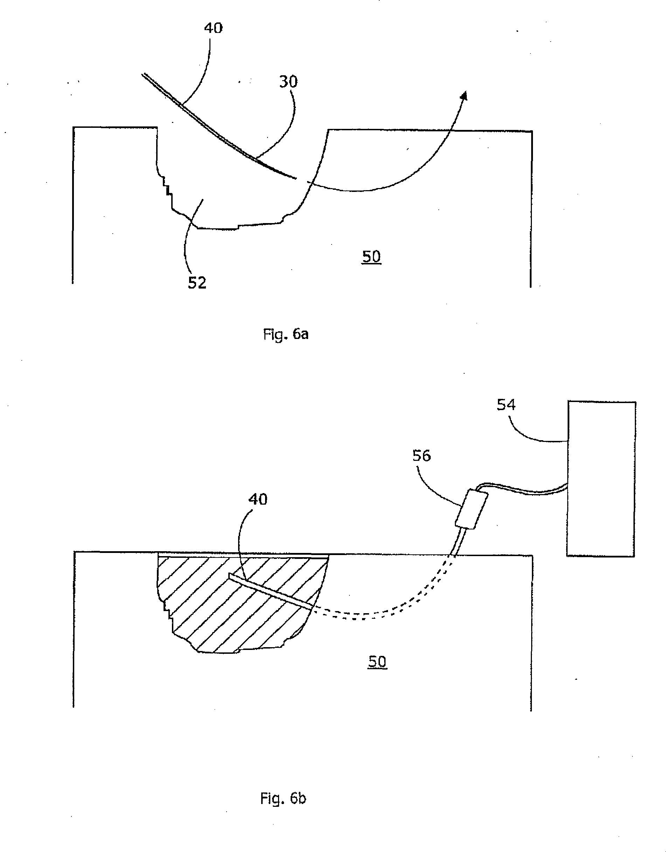

Figures 6a and 6b illustrate a method of use of the system on a patient according to embodiments of the present invention.

A patient 50 has a wound cavity 52 as a result of a surgical procedure. Once the operation is largely complete, an infusion catheter 40 is inserted by use of a surgical needle 30, as described above. The needle 30 will generally be attached to the catheter 40 as part of the manufacturing process, but in some cases may be inserted by the surgeon. The needle 30 is brought into the wound cavity, and then drawn up through the tissue to a point adjacent the wound cavity 52, where it punctures the skin. Through its attachment to the needle 30, the catheter 40 is also pulled through the tissue and out of the skin (Figure 6a).

When the fenestration-bearing segment of the catheter 40 is duly positioned within the wound cavity 52, the catheter 40 may be cut at the non-reinforced region 46. This removes the needle 30 and a small length of catheter, both of which may be discarded. The distal, fenestrated end of the catheter is left in the wound, which may be closed by the surgeon to complete the surgical procedure. The proximal end of the catheter 40 may then be connected to an ambulatory pump 54 via a standard luer twist connector 56 (for example), to provide a prescribed supply of analgesic or other medicament to the wound.

The infusion catheter system according to the present invention has many advantages over conventional systems (such as those described with respect to Figures 2a to 2d). Through its innovative connection method, the needle can take the same diameter as the catheter. Therefore the puncture marks caused in the patient will have a smaller diameter, reducing pain, leakage and the chances of infection. The catheter may be introduced subcutaneously, further reducing the risk of infection by eliminating the risk of skin bacteria being drawn into the wound cavity. This is a far simpler and quicker procedure than conventional methods which employ introducer needles.

It will of course be understood that many variations may be made to the above-described embodiment without departing from the scope of the present invention.

## Claims

1. An infusion catheter system, comprising:
(a) a needle having a tip at one, proximal end and a coupling at the other, distal end;
(b) an infusion catheter comprising a tubular body having a first, proximal end, which is attached to the coupling of the needle, a second, distal end and an internal bore extending between the proximal and distal ends of the tubular body, wherein:
the tubular body includes a reinforced section and a non-reinforced section which allows for easier cutting of the tubular body relative to the reinforced section, thereby allowing for fitting of an infusion pump; and
the tubular body includes an apertured portion which includes apertures in fluid communication with the internal bore.

2. The system of claim 1, wherein the coupling comprises a tubular protrusion and a plurality of barbs which engage the proximal end of the tubular body, optionally the barbs are laser welded to the tubular protrusion, optionally the tubular protrusion has an outer diameter of from about 0.25 mm to about 0.75 mm, optionally from about 0.4 mm to about 0.6 mm.

3. The system of claim 1 or 2, wherein the re-inforced section comprises a major section of the tubular body, optionally a major section of the tubular body which extends from the distal end of the tubular body, the non-reinforced section comprises a section adjacent the coupling, and/or the reinforced section includes a reinforcing element, optionally the reinforcing element comprises a metallic element, optionally the re-inforcing element comprises a wound filament, optionally a helical wire, optionally the tubular body includes a plurality of supports, optionally ribs, which extend along a length of an inner surface of the tubular body and support the reinforcing element to maintain a lumen between an outer surface of the reinforcing element and an inner surface of the tubular body.

4. The system of any of claims 1 to 3, further comprising:
an infusion pump which is attached to the proximal end of the infusion catheter when cut, optionally the infusion pump provides a flow rate of less than 250 ml/hour.

5. The system of any of claims 1 to 4, wherein the apertured portion has a length of from about 50 mm to about 250 mm, optionally (a) the apertured portion has a length of from about 75 mm to about 125 mm, optionally about 100 mm, or (b) the apertured portion has a length of from about 175 mm to about 225 mm, optionally about 200 mm, and/or the apertures are arranged at spaced locations along a length thereof, optionally the apertures have an increasing size in a direction towards the distal end, optionally a progressively increasing size in a direction towards the distal end, optionally the apertures open in different radial directions at the spaced locations along the length of the tubular body, optionally the apertures are located on a spiral, optionally a helical spiral, optionally the apertures are located on a single helical spiral or a pair of helical spirals.

6. The system of any of claims 1 to 5, wherein the apertures are circular and/or the apertures are formed by laser machining.

7. The system of any of claims 1 to 6, wherein the apertures have a dimension of from about 25 µm to about 125 µm.

8. An infusion catheter system, comprising:
an infusion catheter having an internal bore; and
a needle having a tip at one end, and a protrusion extending from the other end;
wherein the protrusion is adapted to mate with the internal bore of the infusion catheter.

9. The system of claim 8, wherein the external diameters of the infusion catheter and the needle are substantially the same, the protrusion is barbed, the diameter of the protrusion is substantially the same as the diameter of the internal bore, the diameters of the protrusion and the internal bore are adapted for an interference fit with one another, the protrusion has an external diameter in the range of from 0.25 mm to 0.75 mm, optionally in the range of from 0.4 mm to 0.6 mm, and/or the infusion catheter is perforated, optionally the perforations are arranged in a helical pattern.

10. The system of claim 8 or 9, wherein the infusion catheter is reinforced with a helically wound wire, optionally the infusion catheter comprises a portion along its length which is free from the helically wound wire.

11. The system of any of claims 8 to 10, further comprising:
a pump for attachment to the infusion catheter.

12. A surgical needle for connection to an infusion catheter, comprising:
a tip at a first end; and
a coupling comprising a tubular protrusion extending from a second end, wherein the protrusion has an external diameter smaller than the diameter of the needle at the second end.

13. The needle of claim 12, wherein the diameter of the protrusion is in the range of from about 0.25 mm to about 0.75 mm, optionally in the range of from about 0.4 mm to about 0.6 mm, the diameter of the needle at the second end is in the range from about 0.75 mm to about 1.25 mm, and/or the coupling further comprises a plurality of barbs, optionally the barbs are laser welded to the tubular protrusion.

14. An infusion catheter, comprising:
a tubular body having a first, proximal end, which is attachable to a coupling of a needle, a second, distal end and an internal bore extending between the proximal and distal ends of the tubular body,
wherein:
the tubular body includes a reinforced section and a non-reinforced section which allows for easier cutting of the tubular body relative to the reinforced section, thereby allowing for fitting to pump apparatus; and
the tubular body includes an apertured portion which includes apertures in fluid communication with the internal bore:

15. The catheter of claim 14, wherein the re-inforced section comprises a major section of the tubular body, optionally a major section of the tubular body which extends from the distal end of the tubular body, the non-reinforced section comprises a section adjacent the proximal end, the reinforced section includes a reinforcing element, optionally the reinforcing element comprises a metallic element, optionally the re-inforcing element comprises a wound filament, optionally a helical wire, optionally the tubular body includes a plurality of supports, optionally internally-protruding ribs, which extend along a length of an inner surface of the tubular body and support the reinforcing element to maintain a lumen between an outer surface of the reinforcing element and an inner surface of the tubular body, the apertured portion has a length of from about 50 mm to about 250 mm, optionally (a) the apertured portion has a length of from about 75 mm to about 125 mm, optionally about 100 mm, or (b) the apertured portion has a length of from about 175 mm to about 225 mm, optionally about 200 mm, the apertures are arranged at spaced locations along a length thereof, optionally the apertures have an increasing size in a direction towards the distal end, optionally a progressively increasing size in a direction towards the distal end, optionally the apertures open in different radial directions at the spaced locations along the length of the tubular body, optionally the apertures are located on a spiral, optionally a helical spiral, optionally the apertures are located on a single helical spiral or a pair of helical spirals, the apertures are circular and/or the apertures are formed by laser machining, and/or the apertures have a dimension of from about 25 µm to about 125 µm.
